# EUROPEAN PATENT SPECIFICATION

(11) **EP 4 037 748 B1**
(45) Date of publication and mention of the grant of the patent: **25.02.2026**
(21) Application number: 20789798.4
(22) Date of filing: 28.09.2020
(51) Int. Cl.: A61M 25/00, A61M 25/06

(54) **COLLAPSIBLE CATHETER**
ZUSAMMENZIEHBARER KATHETER
SONDE POUVANT S'APLATIR

(30) Priority: 30.09.2019 US 201962908199 P
(43) Date of publication of application: 10.08.2022
(73) Proprietor: Abiomed, Inc., Danvers, MA 01923 (US)
(72) Inventor: FANTUZZI, Glen R., Danvers, MA 01923 (US)
(74) Representative: Klunker IP Patentanwälte PartG mbB
(86) International application number: PCT/US2020/053029
(87) International publication number: WO 2021/067169

(56) References cited:
- WO-A1-2008/121888
- WO-A2-2019/055591
- US-A- 5 573 517
- US-A1- 2004 267 308

## Description

### Background:

Physicians performing high risk percutaneous coronary interventions (HRPCI) currently use two separate locations for access into the arterial system if they plan to use mechanical circulatory support. The first access point is for PCI equipment insertion in the right femoral, radial, or brachial arteries, and the second point is for blood pump insertion in the left femoral artery. Using two access points, physicians face several drawbacks including spending additional time performing two insertions, more opportunity for complications during the procedure, more introducers and closure devices required for access, and higher likelihood of inability to treat certain patients like those with vascular issues. Vascular issues such as peripheral artery disease can be present in both femoral arteries, and some scenarios prevent radial or brachial access. Some patients may also require a third access point, for example, if a chronic total occlusion (CTO) must also be treated. The double-access problem also applies to venous situations, wherein, for example, blood pumps are commonly used with pulmonary artery catheterization (PAC), and to large bore procedures, wherein, for example, transcatheter aortic valve replacement (TAVR) or endovascular aneurysm repair (EVAR) require a contralateral contrast injection catheter.

A current technique for performing single-access point intervention involves placing a first device through a peel-away sheath and then advancing a second device next to the first device within the peel-away sheath. Limitations of this technique include inability for the physician to maintain the first device position during second device manipulation, poor hemostasis from the introducer valve which is only intended for one device insertion, and higher likelihood for the peel-away sheath hub to break. In some instances, the introducer sheath valve is pierced with a needle next to the blood pump catheter before inserting the second device; puncturing the sheath valve other than through its center can diminish hemostatic properties of the valve, leading to higher likelihood for complications during surgery such as significant blood loss. In one example, a 14 French (Fr) peel-away sheath is inserted into the vasculature for introduction of a 9 Fr Impella^{®} blood pump and catheter by Abiomed, Inc. Once the pump is up and running, the physician pierces a hole in the hemostatic valve of the introducer sheath using a needle, delivers a wire through the valve, and then delivers a long 7 Fr introducer sheath with a hub for PCI. This example approach is limited, because the 14 Fr peel-away sheath has an inner diameter and breakline, so physicians must carefully account for wall-thickness and remaining available space when inserting the secondary 7 Fr sheath to avoid breaking apart the peel-away sheath.

It would be desirable to have a device allowing for a single-access approach to insert more than one device into the vasculature. A single-access approach has several advantages by dedicating multiple medical devices to one large bore introducer sheath. A single-access system minimizes access to one sterile field and thus reduces the likelihood for contamination, surgical site infection, and other operation-related complications. A single-access system also advantageously reduces the number of access sites to manage and allows for a single, well-defined procedure. In order to conduct insertion of two devices using a single, standard introducer sheath of limited diameter, one approach to a solution would involve reducing the collective size of the devices to at least match the diameter of the sheath.

WO 2019/055591 A2 discloses an integrated sheath assembly for inserting a medical device such as a percutaneous pump into a vessel including a first sheath having a first lumen defining a first opening between proximal and distal ends of the first sheath for passage of a portion of the pump and a second sheath having a second lumen defining a second opening between proximal and distal ends of the second sheath.

### Summary:

According to the invention, an intravascular system comprising the features of claim 1 is provided. The systems and methods (methods not claimed) disclosed herein enable single-access point insertion of multiple medical devices through an introducer sheath into the vasculature of a patient by coupling a medical device to a collapsible catheter. The collapsible catheter, when positioned within a fixed-diameter introducer sheath, allows other medical devices to be passed within the introducer sheath and adjacent to the catheter.

According to the invention, an intravascular system includes an introducer sheath with a fixed inner diameter lumen; a first medical device; and a catheter having: a proximal end, a distal end coupled to the first medical device, and an exterior circumference configured to take first or second dimensions when positioned within the fixed inner diameter lumen. The catheter can be positioned within the lumen of the sheath and leave an annular gap between the exterior circumference of the catheter and the inner circumference of the lumen of the sheath such that the size of the annular gap is variable by virtue of the exterior circumference of the catheter having first and second dimensions. In certain dimensional configurations of the catheter, the annular gap is sized to allow passage of a second medical device therethrough. The first medical device may be a blood pump, and the second medical device may be a PCI device, another catheter, or another introducer sheath. The catheter may be constructed of a flexible layer and a rigid layer removably attached to the catheter, such that the catheter is permitted to vary in size after the rigid layer is removed. The rigid layer may enable a physician or operator to insert and position the catheter within the vasculature. Following removal of the rigid layer, the flexible layer can deform to accommodate the second medical device to fit within the annular gap adjacently. The catheter may alternatively be constructed of a catheter frame that can be actively triggered by a physician to collapse or expand. The catheter frame may be constructed of pluralities of strands, which may form a braided mesh, and a polymer coating. At least one advantage of this exemplary aspect is that the catheter is compatible with commonly used introducer sheaths and medical devices which may be introduced alongside the catheter within the sheath. At least one other advantage of this exemplary aspect is that catheter can be manipulated at the discretion of a physician during positioning of a medical device coupled to the catheter and during introduction of additional medical devices alongside the catheter.

In another aspect, a method (not claimed) for introducing medical devices into the vasculature of a patient through one introducer sheath uses a catheter to accommodate the sizes of multiple devices within the fixed-size of the sheath. The introducer sheath having a fixed inner diameter lumen is inserted percutaneously into the vasculature of a patient to provide an access point for intravascular medical devices. A first medical device, coupled to a catheter, is introduced through the lumen. The first medical device may be a blood pump. While the catheter is positioned within the fixed inner diameter lumen, an exterior circumference of the catheter is configured to adjustably take first or second dimensions. The catheter can be positioned within the lumen of the sheath and leave an annular gap between the exterior circumference of the catheter and the inner circumference of the lumen of the sheath. In certain dimensional configurations of the catheter, the annular gap is sized to allow passage of a second medical device therethrough. The method may further comprise introducing the second medical device through the annular gap. The second medical device may be a PCI device, another catheter, or another introducer sheath. In some implementations, the exterior circumference of the catheter passively changes dimensions as the second device is inserted adjacently. In other implementations, the catheter must be actively triggered by a physician to deform before inserting the second medical device adjacently. In further implementations, the method (not claimed) comprises introducing the introducer sheath into the vasculature, introducing the first medical device coupled to the catheter capable of being triggered to change dimensions, altering the catheter to increase the annular gap, and introducing the second medical device through the annular gap. In some implementations, the second medical device is introduced within the lumen prior to the first medical device coupled to the catheter, and the exterior circumference of the catheter changes dimensions as the catheter is inserted adjacent to the second medical device. At least one advantage of this aspect is that a physician is afforded the ability to manipulate the catheter to their discretion before, during, or after introduction of the catheter through an introducer sheath. At least one other advantage of this aspect is that multiple medical issues can be addressed through one access point by use of multiple medical devices. This aspect may also allow a physician to manipulate the catheter as needed to fit another medical device through the same access point when, for example, another medical device is needed to address a complication that arises during an operation on a patient.

### Brief Description of the Drawings:

The foregoing and other objects and advantages will be apparent upon consideration of the following detailed description, taken in conjunction with the accompanying drawings, in which like reference characters refer to like parts throughout, and in which:
FIG. 1A shows a catheter in a first configuration coupled to a medical device and positioned within a sheath, according to an illustrative implementation;
FIG. 1B shows a catheter in a second configuration coupled to a medical device and positioned within a sheath and another medical device positioned adjacently within the sheath, according to an illustrative implementation;
FIG. 2 shows a catheter having a rigid inner layer and flexible outer layer, according to an illustrative implementation;
FIG. 3 shows a catheter having a flexible inner layer and rigid inner layer, according to an illustrative implementation;
FIG. 4 shows a catheter frame comprising a coated braid for contraction and expansion of a catheter, according to an illustrative implementation;
FIG. 5 shows a flowchart of introduction of a collapsible catheter, according to an illustrative implementation;
FIG. 6 shows a flowchart for introduction of a blood pump coupled to a collapsible catheter and a PCI device through one introducer sheath, according to an illustrative implementation;
FIG. 7 shows a flowchart for introduction of a medical device coupled to a collapsible catheter next to a previously introduced medical device, according to an illustrative implementation; and
FIG. 8 shows a flowchart for introduction of a medical device coupled to a collapsible catheter, altering a configuration of the catheter, and introducing another medical device alongside the catheter, according to an illustrative implementation.

### Detailed Description:

The specification includes references to non-SI units (Fr). These are to be converted to SI-derivative units (mm) using the following factor: 1 mm =3 Fr.

To provide an overall understanding of the systems, method, and devices described herein, certain illustrative embodiments will be described. Although the embodiments and features described herein are specifically described for use in connection with intravascular catheterization, it will be understood that all the components and other features outlined below may be combined with one another in any suitable manner and may be adapted and applied to other types of procedures requiring catheters.

The term proximal should be understood to mean locations on the catheter relatively closer to the operator during use of the catheter, and the term distal should be understood to mean locations on the catheter relatively further away from the operator during use of the catheter. The term upstream should be understood to mean locations on the catheter relatively further upstream in the blood flow within the blood vessel, when the catheter is in place in the patient's blood vessel. The term downstream should be understood to mean locations on the catheter relatively further downstream in the blood flow within the blood vessel, when the catheter is in place in the patient's blood vessel. The term physician should be understood to mean any physician, doctor, or operator in use of the described systems or methods.

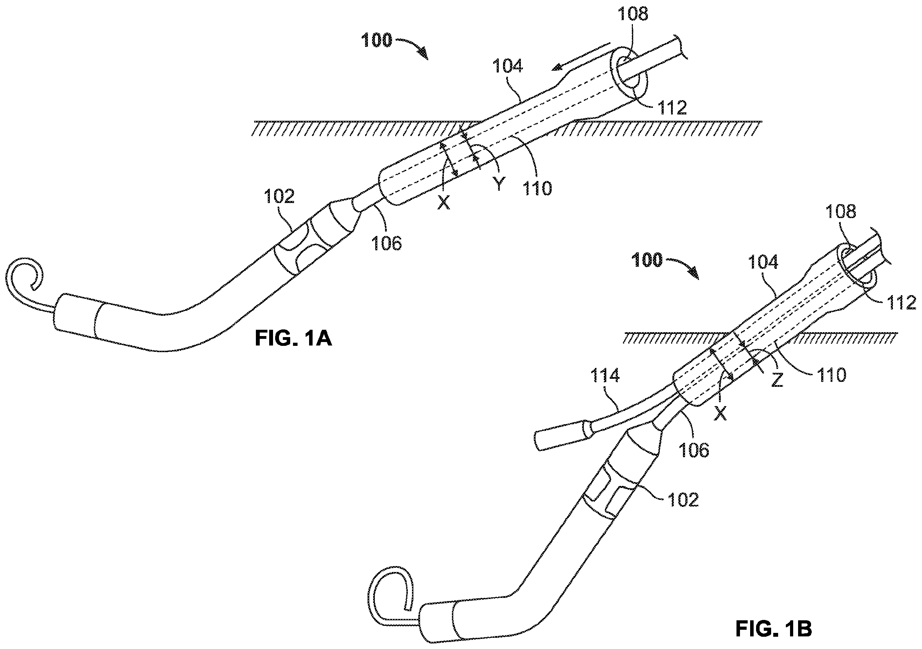

FIGs. 1A and 1B show an intravascular system 100 that is configured for percutaneous insertion of two medical devices into the vasculature of a patient through an introducer sheath. A catheter that is variable in size is coupled to a first medical device, such that a second medical device fit within the introducer sheath alongside the catheter. Cross-sections of the system show the variation in size of the catheter with and without the second medical device introduced.

FIG. 1A shows an intravascular system 100 having a first medical device 102, an introducer sheath 104, and a catheter 106. First medical device 102 is coupled to a distal end of catheter 106. Catheter 106 as shown extends through a lumen 108 of the introducer sheath 104. Lumen 108 has a fixed inner diameter. Introducer sheath 104 has a cross-sectional dimension X. In this arrangement, catheter 106 is in a first configuration and has a first dimension Y. While the catheter 106 is positioned within the lumen 108 of the introducer sheath 104, there exists an annular gap between an exterior circumference 110 of the catheter 106 and an inner circumference 112 of the lumen 108. Catheter 106 has a variable size, and thus the annular gap also has a variable size.

Introducer sheath 104 can be configured to be percutaneously inserted into the vasculature of a patient, and first medical device 102 can be configured to be positioned in the vasculature after being inserted proximally through the introducer sheath 104. In one example, first medical device 102 is a blood pump configured to be positioned within the heart of a patient by way of the vasculature. The blood pump can comprise a motor, a rotor, a pump housing, a cannula, a distal opening, and an atraumatic extension. In some implementations, the first dimension Y is between 8 Fr and 11 Fr. In some implementations, the first dimension Y is between 9 Fr and 10 Fr. In some implementations, the first dimension Y is 9 Fr. In the system of FIG. 1A, the annular gap can have a dimension A1, where *A*1 *= X - Y* in some implementations. For example, dimension Y can have a value that is greater than zero and less than or equal to a value of dimension X, and dimension A1 can have a value in a range from zero to the value of dimension X. At least one advantage of the size-variable catheter 106 is that the annular gap within lumen 108 is also size-variable, and, for example, the size of the annular gap can be tuned to a desired size by changing the configuration of catheter 106, such that a dimension of catheter 106 in a new configuration is not equal to Y. In one example, the desired size of the annular gap can accommodate the size of a second medical device.

FIG. 1B shows the intravascular system 100 having the first medical device 102, the introducer sheath 104, the catheter 106, and a second medical device 114. First medical device 102 is coupled to a distal end of catheter 106. First medical device 102 is positioned in the vasculature after being inserted proximally through the introducer sheath 104. Catheter 106 is positioned within a lumen 108 of the introducer sheath 104. Second medical device 114 is positioned alongside catheter 106 and within the lumen 108 such that it resides within the annular gap formed between the exterior circumference 110 of the catheter 106 and the inner circumference 112 of the lumen 108. While second medical device 114 is simultaneously positioned within the annular gap, catheter 106 is in a second configuration and has a second dimension Z.

Second medical device 114 can be a PCI device, for example. In some implementations, second medical device 114 is a secondary introducer sheath or a secondary catheter. In some implementations, the annular gap in the second configuration is sized to allow passage of the second medical device 114 therethrough. In further implementations, the second medical device 114 cannot fit within the annular gap while the catheter 106 is in the first configuration, so the catheter 106 is in the second configuration to allow passage of the second medical device 114. In some implementations, the annular gap in the second configuration is larger than the annular gap in the first configuration. In some implementations, the catheter 106 is in the second configuration when second medical device 114 passes within the annular gap and in the first configuration when second medical device 114 is not in the annular gap.

In some implementations, the second dimension Z is between 7 Fr and 9 Fr. In some implementations, the second dimension Z is 7 Fr. In the system of FIG. 1B, the annular gap can have dimension A2, where *A*2 *= X - Z* and can have a different value from the dimension of the annular gap in the system of FIG. 1A. For example, second dimension Z can have a value greater than zero and less than or equal to the value of X, and dimension A2 can have a value in a range from zero to the value of X.

Catheter 106 can change from first configuration to second configuration, or vice versa, by active or passive mechanisms comprising, for example, stiffening strands, tensioning wires, braided mesh, sleeves of variable stiffness, rheological materials, inflatable elements, or pneumatic elements. In an implementation of a variable-size catheter having an active mechanism for dimensional change, catheter 106 is triggered by a physician or operator to change configurations. In an implementation of a variable-size catheter having a passive mechanism, introduction of second medical device 114 adjacent to catheter 106 within lumen 108 of introducer sheath 104 forces catheter 106 into the second configuration; the second medical device may exert a normal force against an outer wall of catheter 106 and deform the shape or dimensional configuration of catheter 106.

In certain implementations, only a portion of catheter 106 that is within lumen 108 changes from the first configuration to the second configuration, while the remaining portion(s) of catheter 106 that are not within lumen 108 do not change configuration. For example, first medical device 102 and catheter 106 coupled thereto are introduced through introducer sheath 104 such that a portion of catheter 106 defined by a longitudinal length shorter than an overall longitudinal length of catheter 106 is within lumen 108 of introducer sheath 104, and the remaining length of catheter 106 is positioned outside of introducer sheath 104; as second medical device 114 is introduced alongside catheter 106, only the portion of catheter 106 within lumen 108 undergoes a change from first configuration to second configuration.

In some embodiments, the first dimension and the second dimension are measurements of a length of a cross-section of the catheter. In some embodiments, the cross-section of the catheter is circular, and the first and second dimensions are diameters of the circular cross-section. In some embodiments, the cross-section of the catheter is not circular, and the first and second dimensions are widths of the cross-section in a direction.

In FIG. 1A, catheter 106 is positioned within the lumen 108 of the introducer sheath 104. An annular gap is formed between the exterior circumference 110 of the catheter 106 and the interior circumference 112 of the introducer sheath 104. In this arrangement, catheter 106 is in the first configuration and has first dimension Y, and introducer sheath has cross-sectional dimension X.

In some implementations, the first dimension Y is a measurement of a size of a cross-section of catheter 106. In further implementations, the cross-section is circular, and the first dimension Y is a diameter of the circular cross-section; or the cross-section is not circular, and the first dimension Y is a width of the cross-section in a direction. The cross-section of catheter 106 may be an oval, a circle, or some amorphous form. Alternatively, first dimension Y can be any selected dimension pertaining to the size of catheter 106.

In FIG. 1B, catheter 106 is positioned within the lumen 108 of the introducer sheath 104. Introducer sheath 104 has cross-sectional dimension X. An annular gap is formed between the exterior circumference 110 of the catheter 106 and the interior circumference 112 of the introducer sheath 104. Second medical device 114 is positioned within the annular gap and adjacent to catheter 106. In this arrangement, catheter 106 is in the second configuration and has a second dimension Z, Z being at least smaller than X in order for second medical device 114 to be positioned within the annular gap.

Sheath cross-sectional dimension X sets a limit for the collective size of catheter 106 and second medical device 114 that can fit within a single introducer sheath 104. For example, second medical device 114 can have a cross-sectional dimension S, and dimension X can constrain dimensions Z and S such that *Z + S ≤ X.* In a further example, the sum of the first dimension Y and second device dimension S is greater than sheath dimension X, representing a situation where adjustment of catheter 106 to second dimension Z is necessary for both catheter 106 and second medical device 114 to fit within fixed-inner diameter lumen 108. In some implementations, the second dimension Z is a measurement of a length of a cross-section of catheter 106. In further implementations, the cross-section is circular, and the second dimension Z is a diameter of the circular cross-section; or the cross-section is not circular, and the second dimension Z is a width of the cross-section in a direction. The cross-section of catheter 106 may be an oval, a circle, or some amorphous form. Alternatively, the second dimension Z can be any selected dimension pertaining to the size of catheter 106.

In some implementations, the annular gap while the catheter 106 is in the second configuration is sized to allow a third medical device to fit within the lumen 108, adjacent to the catheter 106 and second medical device 114. In further implementations, the annular gap while the catheter 106 is in the second configuration is sized to allow a plurality of devices to fit within the lumen 108, adjacent to the catheter 106.

At least one advantage of the system shown and described in FIGs. 1A and 1B is that the use of the variable-size catheter 106 allows for second medical device 114 to be positioned alongside catheter 106 within the fixed-diameter lumen 108. By using only one introducer sheath 104 for insertion of both medical devices 102 and 114, the system addresses a need for single-access introduction of multiple intravascular devices. This solution advantageously minimizes complications related to intravascular access and the number of closure devices required to manage access sites.

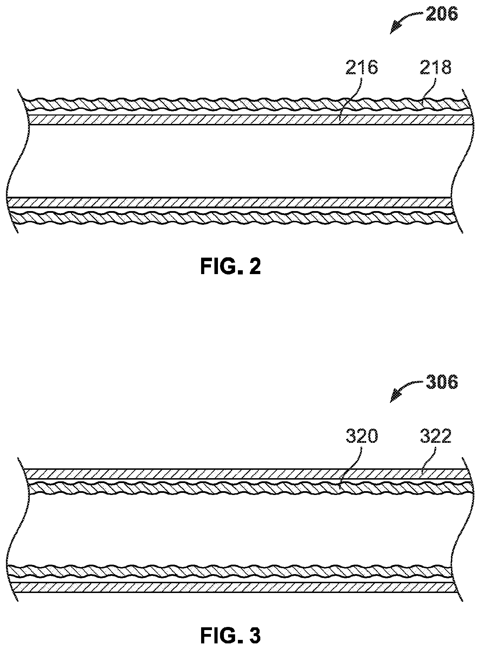

FIG. 2 shows an axial cross-section of a catheter 206 comprising a rigid inner layer 216 and a flexible outer layer 218, catheter 206 being an example of a variable-size catheter such as catheter 106 from FIGs. 1A and 1B. The layers 216 and 218 extend from a proximal end to a distal end of catheter 206. Rigid inner layer 216 is removably attached to catheter 206 or to flexible outer layer 218. Catheter 206 may be catheter 106 in FIGs. 1A and 1B. While catheter 206 is positioned in, for example, the introducer sheath 104 of FIGs. 1A and 1B, the rigid inner layer 216 can be removed proximally. Removal of rigid inner layer 216 allows catheter 206 to change from a first configuration to a second configuration, for example, the first and second configurations described in relation to catheter 106 of FIGs. 1A and 1B. Rigid inner layer 216 is configured to allow a physician to maneuver and manipulate catheter 206 during introduction of a medical device coupled to a distal end of catheter 206, for example, first medical device 102 of FIGs. 1A and 1B.

At least one of rigid inner layer 216 and flexible outer layer 218 can be constructed of a polymer. The polymer can be chosen to have a stiffness suitable for being either rigid or flexible. In some embodiments, the polymer comprises at least one of ethylene vinyl acetate, polyethylene, polyolefin elastomer, maleic anhydride grafted polymer, styrene butadiene styrene, polypropylene-based elastomer, styrene butadiene copolymer, thermoplastic polyester elastomer, thermoplastic polyurethane elastomer, thermoplastic vulcanizate, polyamide, polytetrafluoroethylene, aromatic polyester, polyvinyl chloride, polymethyl methacrylate, acrylonitrile butadiene styrene, styrene acrylonitrile, polystyrene, polyethylene terephthalate copolymer, polycarbonate, polyphenylene oxide, polyphenylene ether, acetate, butyrate, propionate, polysulfone, polyethersulfone, polyarylsulfone, polybutylene terephthalate, polyester terephthalate, acetal, fluorinated ethylene propylene, perfluoroalkoxy polymer, ethylene chlorotrifluoroethylene, polyvinylidene fluoride, tetrafluoroethylene perfluoromethylvinylether, tetrafluoroethylene, hexafluoropropylene, liquid crystal polymer, and polyetheretherkethone.

In some implementations, rigid inner layer 216 comprises at least one peel-away slit which allows for removal of rigid inner layer 216 in a proximal direction. In other implementations, rigid inner layer 216 is removably attached to catheter 206 by a locking mechanism, and rigid inner layer 216 can be removed by twisting a proximal end of rigid inner layer 216 to unlock the mechanism and sliding the rigid inner layer 216 out of catheter 206 proximally. Catheter 206 can be configured to be positioned within an introducer sheath, forming an annular gap between catheter 206 and the introducer sheath. In some implementations, the rigid inner layer 216 is removed from catheter 206, and a second medical device, such as the second medical device 114 in FIGs. 1A and 1B, is introduced adjacent to catheter 206 within a fixed-size lumen, such as lumen 108 in introducer sheath 104 in FIGs. 1A and 1B, and the second medical device exerts a normal force against the flexible outer layer 218, wherein the flexible outer layer is constrained by the normal force to the second configuration such that both the catheter and the medical device fit within the fixed-sized lumen. While rigid inner layer 216 is attached to catheter 206, the catheter 206 does not change from an original configuration, which is be optimal for insertion, maneuvering, and positioning of the catheter 206 and a medical device coupled distally. At least one advantage of catheter 206 is that the difference in stiffness of layers 216 and 218 may allow a physician to manipulate the size of catheter 206 by removing the rigid inner layer 216 during operation such that the remaining flexible outer layer 218 can be deformed to change the size of an annular gap within an introducer sheath in order to fit a second medical device through the annular gap.

FIG. 3 shows an axial cross-section of a catheter 306 comprising a flexible inner layer 320 and a rigid outer layer 322, catheter 306 being an example of a variable-size catheter such as catheter 106 from FIGs. 1A and 1B. The layers 320 and 322 extend from a proximal end to a distal end of catheter 306. Rigid outer layer 322 is removably attached to catheter 306 or to flexible inner layer 320. Catheter 306 may be catheter 106 in FIGs. 1A and 1B. While catheter 306 is positioned in, for example, the introducer sheath 104 of FIGs. 1A and 1B, the rigid outer layer 322 can be removed proximally. Removal of rigid outer layer 322 allows catheter 306 to change from a first configuration to a second configuration, for example, the first and second configurations described in relation to catheter 106 of FIGs. 1A and 1B. Rigid outer layer 322 is configured to allow a physician to maneuver and manipulate catheter 306 during introduction of a medical device coupled to a distal end of catheter 306, for example, first medical device 102 of FIGs. 1A and 1B.

At least one of rigid outer layer 322 and flexible inner layer 320 can be constructed of a polymer. The polymer can be chosen to have a stiffness suitable for being either rigid or flexible. In some implementations, rigid outer layer 322 comprises at least one peel-away slit which allows for removal of rigid outer layer 322 in a proximal direction. In other implementations, rigid outer layer 322 is removably attached to catheter 306 by a locking mechanism, and rigid outer layer 322 can be removed by twisting a proximal end of rigid outer layer 322 to unlock the mechanism and sliding the rigid outer layer 322 out of catheter 306 proximally. Catheter 306 can be configured to be positioned within an introducer sheath, forming an annular gap between catheter 306 and the introducer sheath. In some implementations, the rigid outer layer 322 is removed from catheter 306, and a medical device, such as the second medical device 114 in FIGs. 1A and 1B, is introduced adjacent to catheter 306 within a fixed-size lumen, such as lumen 108 in introducer sheath 104 in FIGs. 1A and 1B, and the medical device exerts a normal force against the flexible inner layer 320, wherein the flexible inner layer is constrained by the normal force to the second configuration such that both the catheter and the medical device fit within the fixed-sized lumen. While rigid outer layer 322 is attached to catheter 306, the catheter 306 does not change from an original configuration, which can be optimal for insertion, maneuvering, and positioning of the catheter 306 and a medical device coupled distally. At least one advantage of catheter 306 is that the difference in stiffness of layers 320 and 322 may allow a physician to manipulate the size of catheter 306 by removing the rigid outer layer 322 during operation such that the remaining flexible inner layer 320 can be deformed to change the size of an annular gap within an introducer sheath in order to fit a second medical device through the annular gap.

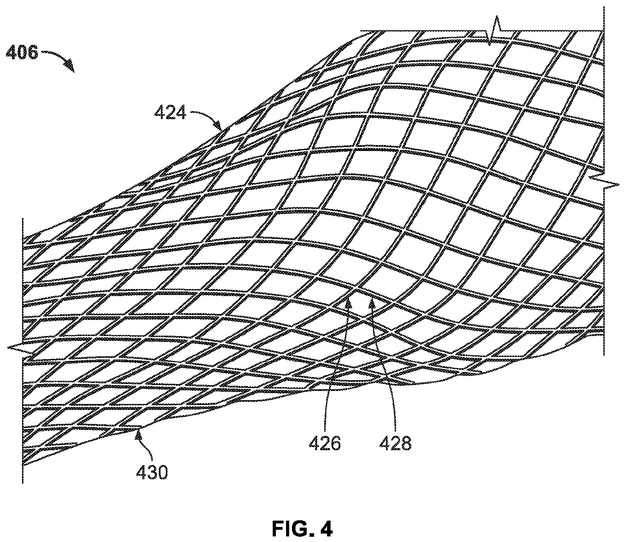

FIG. 4 shows a section of a catheter 406 with a contracted portion and an expanded portion, catheter 406 being an example of a variable size catheter such as catheter 106 from FIGs. 1A and 1B. Contraction and expansion of the catheter 406 is enabled by a catheter frame 424 comprising a first plurality of strands 426 and a second plurality of strands 428, both extending longitudinally from a proximal end to a distal end of catheter 406, and a polymer layer 430 forming a coating about an exterior surface of catheter frame 424. Catheter frame 424 is configured to reversibly deform from a first configuration to a second configuration. Deformation from the first configuration to the second configuration is a contraction of catheter frame 424, and reverse deformation from the second configuration to the first configuration is an expansion of catheter frame 424. First plurality of strands 426 may be wrapped in a spiral direction along the length of the catheter frame 424, and second plurality of strands 428 may be wrapped in a counter-clockwise direction along the length. The first and second pluralities of strands 426 and 428 are configured with a bias to contract or expand, and the strands may be configured to slide relative to each other as catheter frame 424 contracts and expands. Polymer coating 430 is configured to expand and collapse with catheter frame 424 and extends about an interior surface of catheter frame 424.

In some embodiments, the polymer coating 430 comprises at least one of ethylene vinyl acetate, polyethylene, polyolefin elastomer, maleic anhydride grafted polymer, styrene butadiene styrene, polypropylene-based elastomer, styrene butadiene copolymer, thermoplastic polyester elastomer, thermoplastic polyurethane elastomer, thermoplastic vulcanizate, polyamide, polytetrafluoroethylene, aromatic polyester, polyvinyl chloride, polymethyl methacrylate, acrylonitrile butadiene styrene, styrene acrylonitrile, polystyrene, polyethylene terephthalate copolymer, polycarbonate, polyphenylene oxide, polyphenylene ether, acetate, butyrate, propionate, polysulfone, polyethersulfone, polyarylsulfone, polybutylene terephthalate, polyester terephthalate, acetal, fluorinated ethylene propylene, perfluoroalkoxy polymer, ethylene chlorotrifluoroethylene, polyvinylidene fluoride, tetrafluoroethylene perfluoromethylvinylether, tetrafluoroethylene, hexafluoropropylene, liquid crystal polymer, and polyetheretherkethone.

Catheter 406 may be an example of catheter 106 in FIGs. 1A and 1B such that it is coupled to a first medical device and inserted into the vasculature of a patient through an introducer sheath, such as first medical device 102 and introducer sheath 104. In one example, a physician can manipulate the size of catheter 406 by expanding or contracting catheter frame 424 such that an annular gap formed between an external circumference of catheter 406 and an internal circumference of the introducer sheath is also variable in size. Catheter frame 424 may represent an active mechanism for deformation between the first and second configurations. In some implementations, a physician may actively deform catheter 406 by proximally triggering catheter frame 424 to contract or expand. In some implementations, catheter 406 and a first medical device coupled to a distal end of catheter 406 are inserted into the vasculature of a patient through an introducer sheath while the catheter is in a first configuration in which catheter frame 424 is in the expanded state. In further implementations, catheter 406 is triggered by a physician to deform to a second figuration in which the catheter frame 424 is collapsed to the contracted state, wherein the annular gap is enlarged by the deformation. The enlarged annular gap can be sized to allow a second medical device to be inserted within the introducer sheath and adjacent to catheter 406. In some implementations, a blood pump is coupled to a distal end of catheter 406, and the annular gap in the second configuration is sized to allow PCI equipment to be inserted within the same introducer sheath as catheter 406. Catheter frame 424 may comprise a braided mesh formed by the first and second pluralities of strands 426 and 428. In some implementations, catheter frame 424 only comprises the first plurality of strands 426. In further implementations, catheter frame 424 comprises a braided mesh formed by only the first plurality of strands 426. For example, pluralities of strands 426 and 428 can be constructed of polymers, metals, or other materials that have flexible properties. At least one advantage of catheter 406 is that catheter frame 424 can be selectively triggered by a physician to expand or contract, with or without a second medical device being positioned alongside catheter 406. An additional advantage resulting from the design of catheter 406 is a succinct construction of the device that does not necessitate separating catheter 406 into multiple pieces.

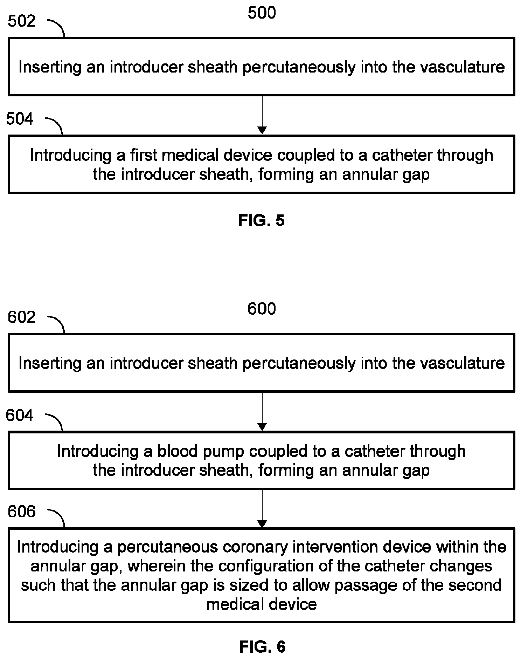

FIG. 5 shows a flowchart describing an exemplary method 500 (not claimed) for introduction of a medical device coupled to a collapsible catheter into the vasculature of a patient through an introducer sheath. Method 500 comprises steps 502 and 504. Step 502 comprises inserting an introducer sheath percutaneously into the vasculature of a patient, the introducer sheath comprising a lumen having a fixed diameter and an inner circumference. Step 504 comprises introducing a first medical device, coupled to a distal end of a catheter, through the introducer sheath. The catheter comprises the distal end, a proximal end, and an exterior circumference sized such that an annular gap is formed between the exterior circumference of the catheter and the inner circumference of the lumen of the introducer sheath when the catheter is positioned within the introducer sheath. The catheter is configured to take first or second configurations when positioned within the fixed diameter lumen, the catheter having a first dimension in the first configuration and a second dimension in the second configuration. For example, this method may be applied to the system shown in FIGs. 1A and 1B, wherein the method would comprise inserting the introducer sheath 104 percutaneously into the vasculature and introducing first medical device 102 coupled to catheter 106 through introducer sheath 104, forming an annular gap between exterior circumference 110 and inner circumference 112. The catheter in method 500 may also be catheters 206, 306, or 406.

The method 500 (not claimed) may further comprise a third step comprising introducing a second medical device within the annular gap, wherein the annular gap in the second configuration is sized to allow passage of the second medical device therethrough. In some implementations, the annular gap in the second configuration is larger than the annular gap in the first configuration. In some implementations of method 500 using catheters 206 or 306 in FIGs. 2 and 3, the catheter can be in the second configuration when the second medical device passes within the annular gap and in the first configuration when the second medical device is not in the annular gap. The first medical device may be a blood pump, and the second medical device may be a PCI device. In other implementations wherein the catheter of method 500 is suited for active contraction and expansion, such as catheter 406 in FIG. 4, the method 500 may further comprise a third step comprising altering the catheter from the first configuration to the second configuration, wherein the annular gap in the second configuration is sized to allow passage of a second medical device therethrough. in implementations of method 500 using catheter 406 from FIG. 4, a physician may trigger the catheter to change its configuration before or after inserting the catheter into the introducer sheath. In further implementations, method 500 further comprises a fourth step comprising introducing the second medical device within the annular gap. At least one advantage of method 500 is that a physician can operate at least two medical devices introduced into the vasculature of a patient through a single introducer sheath, reducing managing and closing of access sites to one well-defined procedure.

FIG. 6 shows a flowchart describing an exemplary method 600 (not claimed) for introduction of a blood pump coupled to a collapsible catheter and a PCI device into the vasculature of a patient through an introducer sheath. Method 600 comprises steps 602, 604, and 606. Step 602 comprises inserting an introducer sheath percutaneously into the vasculature of a patient, the introducer sheath comprising a lumen having a fixed diameter and an inner circumference. Step 604 comprises introducing a blood pump, coupled to a distal end of a catheter, through the introducer sheath, the catheter having proximal and distal ends and an exterior circumference sized such that an annular gap is formed between the exterior circumference of the catheter and the inner circumference of the lumen of the introducer sheath when the catheter is positioned within the introducer sheath. The catheter is configured to take first or second configurations when positioned within the fixed diameter lumen, the catheter having a first dimension in the first configuration and a second dimension in the second configuration. Step 606 comprises introducing a PCI device within the annular gap, the annular gap in the second configuration sized to allow passage of the second medical device therethrough. Method 600 (not claimed) may be applied to the systems shown and described in FIGs. 1-4. For example, the catheter of method 600 can be catheters 106, 206, 306, or 406 being inserted within introducer sheath 104. The change from first configuration to second configuration can be accomplished by passive mechanism, such as described in FIGs. 2-3, or by active mechanism, such as described in FIG. 4. In some implementations of method 600 using catheters 206 or 306 in FIGs. 2 and 3, the catheter is in the second configuration when the second medical device passes within the annular gap and in the first configuration when the PCI device is not in the annular gap. In other implementations wherein the catheter is suited for active contraction and expansion, such as catheter 406 in FIG. 4, the method 600 may further comprise a third step comprising altering the catheter from the first configuration to the second configuration, wherein the annular gap in the second configuration is sized to allow passage of the PCI device therethrough. In implementations of method 600 using catheter 406 from FIG. 4, a physician may trigger the catheter to change its configuration before or after inserting the catheter into the introducer sheath. At least one advantage of method 600 is that a physician may perform high-risk HRPCIs with blood pump support coupled to a variable-size catheter through a single introducer sheath that cannot accommodate the diameter of the PCI device alongside a fixed-size catheter.

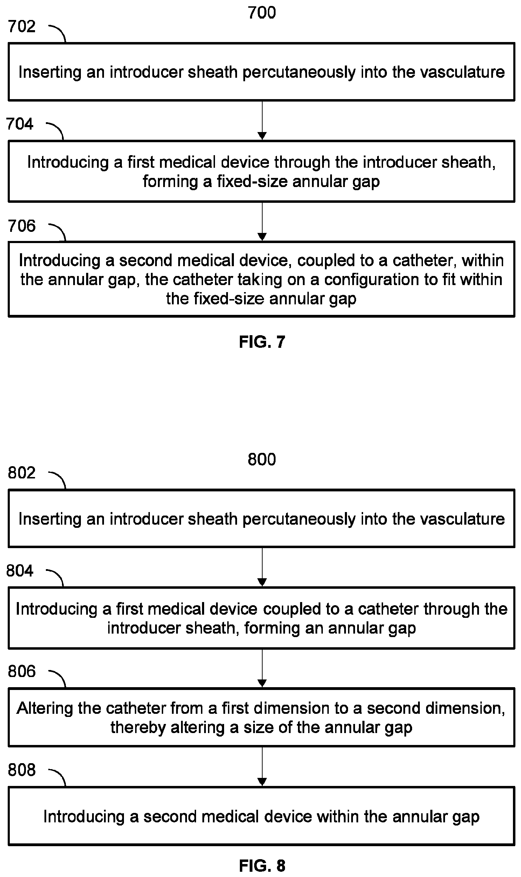

FIG. 7 shows a flowchart describing a method 700 (not claimed) for introduction of a medical device coupled to a collapsible catheter through an introducer sheath already containing a previously inserted medical device. Method 700 comprises steps 702, 704, and 706. Step 702 comprises inserting an introducer sheath percutaneously into the vasculature of a patient, the introducer sheath comprising a lumen having a fixed diameter and an inner circumference. Step 704 comprises introducing a first medical device into the introducer sheath, the first medical device having an exterior circumference sized such that a fixed-size annular gap is formed between the exterior circumference of the catheter and the inner circumference of the lumen of the introducer sheath when the first medical device is positioned within the introducer sheath. Step 706 comprises introducing a second medical device, coupled to a distal end of a catheter, within the annular gap, wherein the catheter is configured to take first or second configurations, the catheter having a first dimension in the first configuration and a second dimension in the second configuration, the second configuration configured to fit within the fixed-sized annular gap.

The devices implemented in method 700 may be those described in FIGs. 1-4, for example, using catheters 106, 206, 306, or 406 and introducer sheath 104. The catheter may passively deform from the first configuration to the second configuration as the catheter is inserted within the fixed-size annular gap. In other implementations, the catheter may be actively triggered to deform to the second configuration sized to fit within the fixed-size annular gap. The first medical device may be a PCI device, and the second medical device may be a blood pump. At least one advantage of method 700 is that the first medical device can be accompanied by the second medical device through the introducer sheath by virtue of the variable-size catheter. For example, a physician may be performing PCI through the introducer sheath, and then a complication may necessitate blood pump support to the PCI, so the physician can introduce a blood pump coupled to the variable-size catheter without opening another access point.

FIG. 8 shows a flowchart describing a method 800 (not claimed) for introduction of a medical device coupled to a collapsible catheter, altering a configuration of the catheter, and introducing another medical device alongside the catheter, according to an illustrative implementation. Method 800 comprises steps 802, 804, 806, and 808. Step 802 comprises inserting an introducer sheath percutaneously into the vasculature, the introducer sheath comprising a lumen having a fixed inner diameter and an inner circumference. Step 804 comprises introducing a first medical device, coupled to a distal end of a catheter, through the introducer sheath, the catheter comprising a proximal end and an exterior circumference having a first dimension such that when the catheter is positioned within the fixed inner diameter lumen, an annular gap exists between the exterior circumference of the catheter and the inner circumference of the sheath lumen. Step 806 comprises altering the exterior circumference of the catheter from the first dimension to a second dimension, such that the annular gap increases to allow passage of a second medical device therethrough. Step 808 comprises introducing the second medical device through the annular gap.

The devices implemented in method 800 may be those described in FIGs. 1A and 1B and 4, for example, using catheters 106 or 406 and introducer sheath 104. The catheter may be actively altered or triggered by a physician to change from having a first dimension to having a second dimension, thus altering the annular gap formed between the catheter and the lumen. In some implementations, the first medical device is a blood pump. In some implementations, the second medical device is a PCI device, a second introducer sheath, or a second catheter. At least one advantage of method 800 is that a physician can selectively alter the size of the catheter while the coupled first medical device is in use within the vasculature of a patient, such that a second medical device can be introduced without opening a second access site into the vasculature. For example, this advantage may be useful if a complication occurs during an operation using the first medical device, and introduction of the second medical device is urgently required by the physician in order to fix the complication.

The foregoing is merely illustrative of the principles of the disclosure, and the apparatuses can be practiced by other than the described embodiments, which are presented for purposes of illustration and not of limitation. It is to be understood that the apparatuses disclosed herein, while shown for use in percutaneous insertion of heart pumps, may be applied to apparatuses in other applications requiring hemostasis.

Variations and modifications will occur to those of skill in the art after reviewing this disclosure. The disclosed features may be implemented, in any combination and subcombination (including multiple dependent combinations and subcombinations), with one or more other features described herein. The various features described or illustrated above, including any components thereof, may be combined or integrated in other systems. Moreover, certain features may be omitted or not implemented.

The systems and methods described may be implemented locally on a heart pump system or a controller of a heart pump system, such as the AIC. The heart pump system may include a data processing apparatus. The systems and methods described herein may be implemented remotely on a separate data processing apparatus. The separate data processing apparatus may be connected directly or indirectly to the heart pump system through cloud applications. The heart pump system may communicate with the separate data processing apparatus in real-time (or near real-time).

In general, embodiments of the subject matter and the functional operations described in this specification can be implemented in digital electronic circuitry, or in computer software, firmware, or hardware, including the structures disclosed in this specification and their structural equivalents, or in combinations of one or more of them.

## Claims

1. An intravascular system (100) for insertion of first and second medical devices (102, 114) into a vasculature of a patient, the system (100) comprising:
an introducer sheath (104) configured to be inserted percutaneously into a vasculature, the introducer sheath (104) comprising a lumen (108) having a fixed inner diameter and an inner circumference;
a first medical device (102); and
a catheter(106; 206; 306; 406) having a proximal end and a distal end, the distal end coupled to the first medical device (102), the catheter (106; 206; 306; 406) having an exterior circumference (110) configured to adjustably take first or second dimensions when positioned within the lumen (108) of the introducer sheath (104), so that the catheter (106; 206; 306; 406) can be positioned within the lumen (108) of the introducer sheath (106; 206; 306; 406) and leave an annular gap between the exterior circumference (110) of the catheter (106; 206; 306; 406) and the inner circumference (112) of the lumen (108) of the introducer sheath (104),
wherein the catheter (106; 206; 306; 406) is configured so that the annular gap while the exterior circumference (110) of the catheter (106; 206; 306; 406) takes the second dimension is sized to allow passage of a second medical device (114) therethrough.

2. The system (100) of claim 1, wherein the catheter (106; 206; 306; 406) is configured so that the annular gap while the exterior circumference (110) of the catheter (106; 206; 306; 406) takes the second dimension is larger than the annular gap while the exterior circumference (110) of the catheter (106; 206; 306; 406) takes the first dimension.

3. The system (100) of claim 1 or 2, wherein exterior circumference (110) of the catheter (106; 206; 306; 406) is configured to take:
the second dimension when the second medical device (114) is within the annular gap; and
the first dimension when the second medical device (114) is not in the annular gap.

4. The system (100) of any one of claims 1-3, wherein the first medical device (102) further comprises or is a blood pump and/or wherein the second medical device (114) comprises or is a percutaneous coronary intervention device.

5. The system (100) of any one of claims 1-4, wherein the catheter (106; 206; 306) comprises an inner layer (216; 320) and an outer layer (218; 322).

6. The system (100) of claim 5, comprising at least one of the following features:
at least one of the inner layer (216; 320) and the outer layer (218; 322) is removably attached to the catheter (106; 206; 306);
the outer layer (218) is flexible and the inner layer (216) is rigid;
the exterior circumference (110) of the catheter (106; 206; 306) is configured to change from the first dimension to the second dimension after removal of the inner layer (216; 320);
the inner layer (320) is flexible and the outer layer (322) is rigid;
the exterior circumference (110) of the catheter (106; 206; 306) is configured to change from the first dimension to the second dimension after removal of the outer layer (218; 322);
the inner layer (216; 320) or the outer layer (218; 322) is configured to be removed in a proximal direction; and
the inner layer (216; 320) or the outer layer (218; 322) comprises at least one peel-away slit which allows for removal of the inner layer (216; 320) or the outer layer (218; 322).

7. The system (100) of any one of claims 1-6, wherein the second medical device (114) exerts a normal force against an outer surface of the catheter (106; 206; 306; 406) while the second medical device (114) is introduced into the introducer sheath (104), the normal force constraining the exterior circumference (110) of the catheter (106; 206; 306; 406) to the second dimension.

8. The system (100) of any one of claims 1-7, wherein the catheter (106; 206; 306; 406) comprises a polymer, wherein preferably the polymer comprises at least one of ethylene vinyl acetate, polyethylene, polyolefin elastomer, maleic anhydride grafted polymer, styrene butadiene styrene, polypropylene-based elastomer, styrene butadiene copolymer, thermoplastic polyester elastomer, thermoplastic polyurethane elastomer, thermoplastic vulcanizate, polyamide, polytetrafluoroethylene, aromatic polyester, polyvinyl chloride, polymethyl methacrylate, acrylonitrile butadiene styrene, styrene acrylonitrile, polystyrene, polyethylene terephthalate copolymer, polycarbonate, polyphenylene oxide, polyphenylene ether, acetate, butyrate, propionate, polysulfone, polyethersulfone, polyarylsulfone, polybutylene terephthalate, polyester terephthalate, acetal, fluorinated ethylene propylene, perfluoroalkoxy polymer, ethylene chlorotrifluoroethylene, polyvinylidene fluoride, tetrafluoroethylene perfluoromethylvinylether, tetrafluoroethylene, hexafluoropropylene, liquid crystal polymer, and polyetheretherkethone.

9. The system (100) of any one of claims 1-4, wherein the catheter (106; 406) comprises a catheter frame (424) configured to reversibly deform the exterior circumference (110) of the catheter (106; 406) from the first dimension to the second dimension.

10. The system (100) of claim 9, further comprising at least one of the following features:
a polymer layer (430) covering an outer circumference of the catheter frame (424) and forming a coating about an exterior surface of the catheter frame (424), wherein the coating preferably extends about an interior surface of the catheter frame (424);
the catheter frame (424) is configured to contract and expand, wherein contraction of the catheter frame (424) comprises a change from the first dimension to the second dimension and expansion of the catheter frame (424) comprises a change from the second dimension to the first dimension, wherein the polymer layer (430) is configured to expand and collapse with the catheter frame (424);
the catheter (106; 406) comprises a plurality of strands (426; 428) configured with a bias to contract or expand and extending longitudinally between the proximal end and the distal end, wherein the catheter (106; 406) is preferably configured to permit the strands (426; 428) to slide relative to each other when the catheter frame (424) contracts and expands; and
the catheter frame (424) comprises a braided mesh formed of a first plurality of strands (426), the first plurality of strands (426) being wrapped in a spiral direction along a length of the catheter frame (424), wherein the catheter frame (424) preferably comprises a second plurality of strands (428), the second plurality of strands (428) being wrapped in a counter-clockwise direction along the length.

11. The system (100) of any one of claims 1-10, wherein:
the first dimension is between about 3 mm (9 Fr) and about 3.33 mm (10 Fr); and
the second dimension is greater than about 2.33 mm (7 Fr) and less than about 3 mm (9 Fr).

12. The system (100) of any one of claims 1-11, wherein a first portion of the catheter (106; 206; 306; 406) is within the lumen (108) of the introducer sheath (104) and a second portion of the catheter (106; 206; 306; 406) is not within the lumen (108)of the introducer sheath (104), wherein the first portion of the catheter (106; 206; 306; 406) preferably changes from the first dimension to the second dimension while the second portion of the catheter (106; 206; 306; 406) remains in the first dimension.

13. The system (100) of any one of claims 1-12, wherein the second dimension allows the catheter (106; 206; 306; 406) and the second medical device (114) to fit within the lumen (108) of the introducer sheath (104) and adjacent to each other.

14. The system (100) of any one of claims 1-13, wherein the first dimension and the second dimension are measurements of a length of a cross-section of the catheter (106; 206; 306; 406).

15. The system (100) of claim 14, wherein the cross-section of the catheter (106; 206; 306; 406) is circular and the first and second dimensions are diameters of the circular cross-section, or wherein the cross-section of the catheter (106; 206; 306; 406) is not circular and the first and second dimensions are widths of the cross-section in a direction.

## Patentansprüche

1. Intravaskuläres System (100) zum Einführen einer ersten und einer zweiten medizinischen Vorrichtung (102, 114) in ein Gefäßsystem eines Patienten, wobei das System (100) aufweist:
eine Einführungshülse (104), die so konfiguriert ist, dass sie perkutan in ein Gefäßsystem eingeführt werden kann, wobei die Einführungshülse (104) ein Lumen (108) mit einem festen Innendurchmesser und einem Innenumfang aufweist;
eine erste medizinische Vorrichtung (102); und
einen Katheter (106; 206; 306; 406) mit einem proximalen Ende und einem distalen Ende, wobei das distale Ende mit der ersten medizinischen Vorrichtung (102) gekoppelt ist, wobei der Katheter (106; 206; 306; 406) einen Außenumfang (110) aufweist, der so konfiguriert ist, dass er anpassbare erste oder zweite Abmessungen annimmt, wenn er innerhalb des Lumens (108) der Einführungshülse (104) angeordnet ist, sodass der Katheter (106; 206; 306; 406) innerhalb des Lumens (108) der Einführungshülse (106; 206; 306; 406) angeordnet werden kann und einen ringförmigen Spalt zwischen dem Außenumfang (110) des Katheters (106; 206; 306; 406) und dem Innenumfang (112) des Lumens (108) der Einführungshülse (104) lassen kann,
wobei der Katheter (106; 206; 306; 406) so konfiguriert ist, dass der ringförmige Spalt, während der Außenumfang (110) des Katheters (106; 206; 306; 406) die zweite Abmessung einnimmt, so dimensioniert ist, dass eine zweite medizinische Vorrichtung (114) hindurchgeführt werden kann.

2. System (100) nach Anspruch 1, wobei der Katheter (106; 206; 306; 406) so konfiguriert ist, dass der ringförmige Spalt, während der Außenumfang (110) des Katheters (106; 206; 306; 406) die zweite Abmessung annimmt, größer ist als der ringförmige Spalt, während der Außenumfang (110) des Katheters (106; 206; 306; 406) die erste Abmessung einnimmt.

3. System (100) nach Anspruch 1 oder 2, wobei der Außenumfang (110) des Katheters (106; 206; 306; 406) so konfiguriert ist, dass er die zweite Abmessung annimmt, wenn sich die zweite medizinische Vorrichtung (114) innerhalb des ringförmigen Spalts befindet, und die erste Abmessung annimmt, wenn sich die zweite medizinische Vorrichtung (114) nicht im ringförmigen Spalt befindet.

4. System (100) nach einem der Ansprüche 1 bis 3, wobei die erste medizinische Vorrichtung (102) weiter eine Blutpumpe aufweist oder eine Blutpumpe ist und/oder wobei die zweite medizinische Vorrichtung (114) eine perkutane

5. System (100) nach einem der Ansprüche 1 bis 4, wobei der Katheter (106; 206; 306) eine innere Schicht (216; 320) und eine äußere Schicht (218; 322) aufweist.

6. System (100) nach Anspruch 5, das mindestens eines der folgenden Merkmale aufweist:
mindestens eine der inneren Schicht (216; 320) und der äußeren Schicht (218; 322) ist entfernbar an dem Katheter befestigt (106; 206; 306);
die äußere Schicht (218) ist flexibel und die innere Schicht (216) ist starr;
der Außenumfang (110) des Katheters (106; 206; 306) ist so konfiguriert, dass er sich nach dem Entfernen der inneren Schicht (216; 320) von der ersten Dimension in die zweite Dimension verändert;
die innere Schicht (320) ist flexibel und die äußere Schicht (322) ist starr;
der Außenumfang (110) des Katheters (106; 206; 306) ist so konfiguriert, dass er sich nach dem Entfernen der äußeren Schicht (218; 322) von der ersten Dimension in die zweite Dimension verändert;
die innere Schicht (216; 320) oder die äußere Schicht (218; 322) ist so konfiguriert, dass sie in einer proximalen Richtung entfernt werden kann; und
die innere Schicht (216; 320) oder die äußere Schicht (218; 322) weist mindestens einen abziehbaren Schlitz auf, der ein Entfernen der inneren Schicht (216; 320) oder der äußeren Schicht (218; 322) ermöglicht.

7. System (100) nach einem der Ansprüche 1 bis 6, wobei die zweite medizinische Vorrichtung (114) eine Normalkraft gegen eine Außenoberfläche des Katheters (106; 206; 306; 406) ausübt, während die zweite medizinische Vorrichtung (114) in die Einführungshülse (104) eingeführt wird, wobei die Normalkraft den Außenumfang (110) des Katheters (106; 206; 306; 406) auf die zweite Dimension begrenzt.

8. System (100) nach einem der Ansprüche 1 bis 7, wobei der Katheter (106; 206; 306; 406) ein Polymer aufweist, wobei das Polymer vorzugsweise mindestens eines der folgenden aufweist: Ethylenvinylacetat, Polyethylen, Polyolefinelastomer, Maleinsäureanhydrid-gepfropftem Polymer, Styrol-Butadien-Styrol, Elastomer auf Polypropylenbasis, Styrol-Butadien-Copolymer, thermoplastischem Polyesterelastomer, thermoplastischem Polyurethanelastomer, thermoplastischem Vulkanisat, Polyamid, Polytetrafluorethylen, aromatischem Polyester, Polyvinylchlorid, Polymethylmethacrylat, Acrylnitril-Butadien-Styrol, Styrol-Acrylnitril, Polystyrol, Polyethylenterephthalat-Copolymer, Polycarbonat, Polyphenylenoxid, Polyphenylenether, Acetat, Butyrat, Propionat, Polysulfon, Polyethersulfon, Polyarylsulfon, Polybutylenterephthalat, Polyesterterephthalat, Acetal, fluoriertes Ethylenpropylen, Perfluoralkoxypolymer, Ethylenchlorotrifluorethylen, Polyvinylidenfluorid,

9. System (100) nach einem der Ansprüche 1 bis 4, wobei der Katheter (106; 406) einen Katheterrahmen (424) aufweist, der so konfiguriert ist, dass er den Außenumfang (110) des Katheters (106; 406) reversibel von der ersten Dimension in die zweite Dimension verformt.

10. System (100) nach Anspruch 9, das weiter mindestens eines der folgenden Merkmale aufweist:
eine Polymerschicht (430), die einen Außenumfang des Katheterrahmens (424) bedeckt und eine Beschichtung um eine Außenoberfläche des Katheterrahmens (424) bildet, wobei sich die Beschichtung vorzugsweise um eine Innenfläche des Katheterrahmens (424) erstreckt;
der Katheterrahmen (424) so konfiguriert ist, dass er sich zusammenzieht und
ausdehnt, wobei das Zusammenziehen des Katheterrahmens (424) eine Änderung von der ersten Dimension zur zweiten Dimension aufweist und das Ausdehnen des Katheterrahmens (424) eine Änderung von der zweiten Dimension zur ersten Dimension aufweist, wobei die Polymerschicht (430) so konfiguriert ist, dass sie sich mit dem Katheterrahmen (424) ausdehnt und zusammenfällt;
der Katheter (106; 406) eine Vielzahl von Strängen (426; 428) aufweist, die mit einer Vorspannung zum Zusammenziehen oder Ausdehnen konfiguriert sind und sich in Längsrichtung zwischen dem proximalen Ende und dem distalen Ende erstrecken, wobei der Katheter (106; 406) vorzugsweise so konfiguriert ist, dass die Stränge (426; 428) relativ zueinander gleiten können, wenn sich der Katheterrahmen (424) zusammenzieht und ausdehnt; und
der Katheterrahmen (424) ein geflochtenes Netz aufweist, das aus einer ersten Vielzahl von Strängen (426) gebildet ist, wobei die erste Vielzahl von Strängen (426) in einer spiralförmigen Richtung entlang einer Länge des Katheterrahmens (424) gewickelt ist, wobei der Katheterrahmen (424) vorzugsweise eine zweite Vielzahl von Strängen (428) aufweist, wobei die zweite Vielzahl von Strängen (428) in einer gegen den Uhrzeigersinn gerichteten Richtung entlang der Länge gewickelt ist.

11. System (100) nach einem der Ansprüche 1 bis 10, wobei:
die erste Abmessung zwischen etwa 3 mm (9 Fr) und etwa 3,33 mm (10 Fr) liegt; und
die zweite Abmessung größer als etwa 2,33 mm (7 Fr) und kleiner als etwa 3 mm (9 Fr) ist.

12. System (100) nach einem der Ansprüche 1 bis 11, wobei sich ein erster Abschnitt des Katheters (106; 206; 306; 406) innerhalb des Lumens (108) der Einführungshülse (104) befindet und ein zweiter Abschnitt des Katheters (106; 206; 306; 406) sich nicht innerhalb des Lumens (108) der Einführungshülse (104) befindet, wobei der erste Abschnitt des Katheters (106; 206; 306; 406) vorzugsweise von der ersten Dimension

13. System (100) nach einem der Ansprüche 1 bis 12, wobei die zweite Abmessung es dem Katheter (106; 206; 306; 406) und der zweiten medizinischen Vorrichtung (114) ermöglicht, in das Lumen (108) der Einführungshülse (104) und nebeneinander zu passen.

14. System (100) nach einem der Ansprüche 1 bis 13, wobei die erste Dimension und die zweite Dimension Längenmaße eines Querschnitts des Katheters (106; 206; 306; 406) sind.

15. System (100) nach Anspruch 14, wobei der Querschnitt des Katheters (106; 206; 306; 406) kreisförmig ist und die erste und zweite Abmessung Durchmesser des kreisförmigen Querschnitts sind, oder wobei der Querschnitt des Katheters (106; 206; 306; 406) nicht kreisförmig ist und die erste und zweite Abmessung Breiten des Querschnitts in einer Richtung sind.

## Revendications

1. Système intravasculaire (100) pour introduction de premier et second dispositifs médicaux (102, 114) dans une vasculature d'un patient, le système (100) comprenant :
une gaine d'introducteur (104) configurée pour être introduite de manière percutanée dans une vasculature, la gaine d'introducteur (104) comprenant une lumière (108) présentant un diamètre intérieur fixe et une circonférence intérieure ;
un premier dispositif médical (102), et
un cathéter (106; 206; 306; 406) présentant une extrémité proximale et une extrémité distale, l'extrémité distale étant accouplée avec le premier dispositif médical (102), le cathéter (106; 206; 306; 406) présentant une circonférence extérieure (110) configurée pour adopter, de manière réglable, des première ou seconde dimensions lorsque positionné dans la lumière (108) de la gaine d'introducteur (104), de sorte que le cathéter (106; 206; 306; 406) peut être positionné dans la lumière (108) de la gaine d'introducteur (106; 206; 306; 406) et laisser un espace annulaire entre la circonférence extérieure (110) du cathéter (106; 206; 306; 406) et la circonférence intérieure(112) de la lumière (108) de la gaine d'introducteur (104),
dans lequel le cathéter (106; 206; 306; 406) est configuré de manière à ce que tandis que la circonférence extérieure (110) du cathéter (106; 206; 306; 406) prend la seconde dimension, l'espace annulaire a une taille permettant le passage à travers lui d'un second dispositif médical (114).

2. Système (100) la revendication 1, dans lequel le cathéter (106; 206; 306; 406) est configuré de manière à ce que, tandis que la circonférence extérieure (110) du cathéter (106; 206; 306; 406) prend la seconde dimension, l'espace annulaire est plus grand que l'espace annulaire tandis que la circonférence extérieure (110) du cathéter (106; 206; 306; 406) adopte la première dimension.

3. Système (100) la revendication 1 ou 2, dans lequel la circonférence extérieure (110) du cathéter (106; 206; 306; 406) est configurée de manière à adopter :
la seconde dimension lorsque le second dispositif médical (114) est dans l'espace annulaire, et
la première dimension lorsque le second dispositif médical (114) n'est pas dans l'espace annulaire.

4. Système (100) selon l'une quelconque des revendications 1 à 3, dans lequel en outre, le premier dispositif médical (102) comprend, ou est, une pompe sanguine, et/ou dans lequel le second dispositif médical (114) comprend, ou est, un dispositif d'intervention coronaire percutané.

5. Système (100) selon l'une quelconque des revendications 1 à 4, dans lequel le cathéter (106; 206; 306) comprend une couche intérieure (216; 320) et une couche extérieure (218; 322).

6. Système (100) la revendication 5, comprenant au moins une des caractéristiques suivantes :
au moins une couche parmi la couche intérieure (216; 320), et la couche extérieure (218; 322) est fixée de manière amovible sur le cathéter (106; 206; 406) ;
la couche extérieure (218) est flexible, et la couche intérieure (216) est rigide ;
la circonférence extérieure (110) du cathéter (106; 206; 306) est configurée pour passer de la première dimension à la seconde dimension après retrait de la couche intérieure (216; 230) ;
la couche intérieure (320) est flexible, et la couche extérieure (322) est rigide ;
la circonférence extérieure (110) du cathéter (106; 206; 306) est configurée pour passer de la première dimension à la seconde dimension après retrait de la couche extérieure (218; 322) ;
la couche intérieure (216; 320) ou la couche extérieure (218; 322) est configurée pour être retirée dans une direction proximale, et
la couche intérieure (216; 320) ou la couche extérieure (218; 322) comprend au moins une fente pelable, laquelle permet le retrait de la couche intérieure (216; 320) ou de la couche extérieure (218; 322).

7. Système (100) selon l'une quelconque des revendications 1 à 6, dans lequel le second dispositif médical (114) exerce une force normale contre une surface extérieure du cathéter (106; 206; 306; 406) tandis que le second dispositif médical (114) est introduit dans la gaine d'introducteur (104), la force normale contraignant la circonférence extérieure (110) du cathéter (106; 206; 306; 406) sur la seconde dimension.

8. Système (100) selon l'une quelconque des revendications 1 à 7, dans lequel le cathéter (106; 206; 306; 406) comprend un polymère, et dans lequel de préférence, le polymère comprend au moins un élément suivant : l'acétate de vinyle-éthylène, le polyéthylène, un élastomère de polyoléfine, un polymère greffé à l'anhydride maléique, du styrène-butadiène-styrène, un élastomère à base de polypropylène, un copolymère de styrène-butadiène, un élastomère de polyester thermoplastique, un élastomère de polyuréthane thermoplastique, un vulcanisat thermoplastique, du polyamide, du polytétrafluoroéthylène, un polyester aromatique, du chlorure de polyvinyle, un méthacrylate de polyméthyle, de l'acrylonitrile-butadiène-styrène, de l'acrylonitrile-styrène, du polystyrène, un copolymère de polyéthylène téréphtalate, du polycarbonate, de l'oxyde de polyphénylène, du polyphénylène éther, de l'acétate, du butyrate, du propionate, la polysulfone, la polyéthersulfone, la polyarylsulfone, le polybutylène téréphtalate, le polyester téréphtalate, l'acétal, l'éthylène-propylène fluoré, un polymère perfluoroalkoxy, un éthylène chlorotrifluoroéthylène, du fluorure de polyvinylidène, un tétrafluoroéthylène-perfluorométhylvinyléther, le tétrafluoroéthylène, l'hexafluoropropylène, un polymère de cristaux liquides, et la polyétheréthercétone.

9. Système (100) selon l'une quelconque des revendications 1 à 4, dans lequel le cathéter (106; 406) comprend un cadre de cathéter (424) configuré pour déformer de manière réversible la circonférence extérieure (110) du cathéter (106; 406) de la première dimension vers la seconde dimension.

10. Système (100) la revendication 9, comprenant en outre au moins une des caractéristiques suivantes :
une couche polymère (430) couvrant une circonférence extérieure du cadre de cathéter (424) et formant un revêtement autour d'une surface extérieure du cadre de cathéter (424), dans lequel le revêtement s'étend de préférence autour d'une surface intérieure du cadre de cathéter (424) ;
le cadre de cathéter (424) est configuré pour se contracter et se déployer, dans lequel la contraction du cadre de cathéter (424) comprend un passage de la première dimension à la seconde dimension, et le déploiement du cadre de cathéter (424) comprend un passage de la seconde dimension à la première dimension, dans lequel la couche polymère (430) est configurée pour se déployer et s'escamoter avec le cadre de cathéter (424) ;
le cathéter (106; 406) comprend une pluralité de brins (426, 428) configurée avec une sollicitation pour se contracter ou se dilater et s'étendant longitudinalement entre l'extrémité proximale et l'extrémité distale, et dans lequel le cathéter (106 ; 406) est de préférence configuré pour permettre aux brins (426 ; 428) de coulisser les uns par rapport aux autres lorsque le cadre de cathéter (424) se contracte et se déploie, et
le cadre de cathéter (424) comprend un maillage tressé formé d'une première pluralité de brins (426), la première pluralité de brins (426) étant enveloppée suivant une direction spiralée sur une longueur du cadre de cathéter (424), et dans lequel le cadre de cathéter (424) comprend de préférence une seconde pluralité de brins (428), la seconde pluralité de brins (428) étant enveloppée dans une direction antihoraire sur la longueur.

11. Système (100) selon l'une quelconque des revendications 1 à 10, dans lequel :
la première dimension est comprise entre environ 3 mm (9 Fr) et environ 3,33 mm (10 Fr), et
la seconde dimension est supérieure à environ 2,33 mm (7 Fr) et inférieure à environ 3 mm (9 Fr).

12. Système (100) selon l'une quelconque des revendications 1 à 11, dans lequel une première portion du cathéter (106; 206; 306; 406) est dans la lumière (108) de la gaine d'introducteur (104), et une seconde portion du cathéter (106; 206; 306; 406) n'est pas dans la lumière (108) de la gaine d'introducteur (104), dans lequel la première portion du cathéter (106; 206; 306; 406) passe de préférence de la première dimension à la seconde dimension tandis que la seconde portion du cathéter (106; 206; 306; 406) reste dans la première dimension.

13. Système (100) selon l'une quelconque des revendications 1 à 12, dans lequel la seconde dimension permet au cathéter (106; 206; 306; 406) et au second dispositif médical (114) de s'adapter dans la lumière (108) de la gaine d'introducteur (104) de manière adjacente l'un avec l'autre.

14. Système (100) selon l'une quelconque des revendications 1 à 13, dans lequel la première dimension et la seconde dimension sont des mesures d'une longueur d'une section du cathéter (106; 206; 306; 406).

15. Système (100) la revendication 14, dans lequel la section du cathéter (106; 206; 306; 406) est circulaire, et les première et seconde dimensions sont des diamètres de section circulaire, ou dans lequel la section du cathéter (106; 206; 306; 406) n'est pas circulaire, et les première et seconde dimensions sont des largeurs de la section dans une direction.
